# EUROPEAN PATENT SPECIFICATION

(11) **EP 1 382 346 B1**
(45) Date of publication and mention of the grant of the patent: **26.09.2007**
(21) Application number: 03018701.7
(22) Date of filing: 21.03.1997
(51) Int. Cl.: A61K 35/00, A61K 35/12, C12N 11/04

(54) **Conditioned medium and diffusible biological product produced by incubating entrapped cells**
Konditioniertes Medium und diffusionsfähiges biologisches Material gewonnen durch Inkubierung von eingeschlossenen Zellen
Milieu conditionné et produit biologique diffusible obtenu par incubation de cellules piégées

(30) Priority: 03.04.1996 US 625595; 07.11.1996 US 745063
(43) Date of publication of application: 21.01.2004
(62) Divisional of application: 97915180.0
(73) Proprietor: THE ROGOSIN INSTITUTE, New York, NY 10021 (US)
(72) Inventor: Jain, Kanti, Alpine, NJ 07260 (US); Rubin, Albert, Englewood, NJ 07631 (US); Asina, Shirin, New York, NY 10021 (US); Smith, Barry, New York, NY 10021 (US); Stenzal, Kurt, New York, NY 10021 (US)
(74) Representative: Weickmann, Franz Albert

(56) References cited:
- WO-A-93/22346
- WO-A-95/19430
- WO-A-95/28480
- DATABASE WPI Section Ch, Week 198948 Derwent Publications Ltd., London, GB; Class B04, AN 1989-353217 XP002256191 & JP 01 265029 A (KOKEN KK), 23 October 1989 (1989-10-23)

## Description

The present invention relates to the encapsulation of cells in agarose and collagen, followed by coating with agarose, therapeutic methods employing the materials cells, and manufacture thereof.

### Background and Prior Art

The encapsulation of various biological materials in biologically compatible materials is a technique that has been used for some time, albeit with limited success. Exemplary of the art are U.S. Patent Nos. 5,227,298 (Weber, et al); 5,053,332 (Cook, et al); 4,997,443 (Walthall, et al); 4,971,833 (Larsson, et al); 4,902,295 (Walthall, et al); 4,798,786 (Tice, et al); 4,673,566 (Goosen, et al); 4,647,536 (Mosbach, et al); 4,409,331 (Lim); 4,392,909 (Lim); 4,352,883 (Lim); and 4,663,286 (Tsang, et al). Also of interest is copending application Serial No. 08/483,738, filed June 7, 1995 to Jain, et al, Jain, et al discusses, in some detail, the encapsulation of secretory cells in various bio-compatible materials. As discussed therein, secretory cells are cells which secrete biological products. Generally, secretory cells possess at least some properties of endocrine cells, and may generally be treated as equivalent to cells which are endocrine in nature. The copending application discusses, e.g., the encapsulation of insulin producing cells, preferably in the form of islets, into agarose-collagen beads which have also been coated with agarose. The resulting products are useful in treating conditions where a subject needs insulin therapy, such as diabetes.

The Jain, et al application discusses in some detail, the prior approaches taken by the art in transplantation therapy. These are summarized herein as well.

Five major approaches to protecting the transplanted tissue from the host's immune response are known. All involve attempts to isolate the transplanted tissue from the host's immune system. The immunoisolation techniques used to date include: extravascular diffusion chambers, intravascular diffusion chambers, intravascular ultrafiltration chambers, microencapsulation, and macroencapsula-tion. All of these methods have failed, however, due to one or more of the following problems: a host fibrotic response to the implant material, instability of the implant material, limited nutrient diffusion across semi-permeable membranes, secretagogue and product permeability, and diffusion lag-time across semi-permeable membrane barriers.

For example, a microencapsulation procedure for enclosing viable cells, tissues, and other labile membranes within a semipermeable membrane was developed by Lim in 1978. (Lim, Research report to Damon Corporation (1978)). Lim used microcapsules of alginate and poly L-lysine to encapsulate the islets of Langerhans. In 1980, the first successful *in vivo* application of this novel technique in diabetes research was reported (Lim, et al., Science 210: 908 (1980)). The implantation of these microencapsulated islets of Langerhans resulted in sustaining a euglycemic state in diabetic animals. Other investigators, however, repeating these experiments, found the alginate to cause a tissue reaction and were unable to reproduce Lim et al's results (Lamberti, et al. Applied Biochemistry and Biotechnology 10: 101 (1984); Dupuy, et al., J. Biomed. Material and Res. 22: 1061 (1988); Weber, et al., Transplantation 49: 396 (1990); and Doon-shiong, et al., Transplantation Proceedings 22: 754 (1990)). The water solubility of these polymers is now considered to be responsible for the limited stability and biocompatibility of these microcapsules *in vivo* (Dupuy, et al. *supra,* Weber et al. *supra,* Doon-shiong, et al., *supra,* and Smidsrod, Faraday Discussion of Chemical Society 57: 263 (1974)).

Recently, Iwata et al., (Iwata, et al. Jour. Biomedical Material and Res. 26: 967 (1992)) utilized agarose for microencapsulation of allogeneic pancreatic islets and discovered that it could be used as a medium for the preparation of microbeads. In their study, 1500-2000 islets were micro- encapsulated individually in 5% agarose and implanted into streptozotocin-induced diabetic mice. The graft survived for a long period of time, and the recipients maintained normoglycemia indefinitely.

Their method, however, suffers from a number of drawbacks. It is cumbersome and inaccurate. For example, many beads remain partially coated and several hundred beads of empty agarose form. Additional time is thus required to separate encapsulated islets from empty beads. Moreover, most of the implanted microbeads gather in the pelvic cavity, and a large number of islets are required in completely coated individual beads to achieve normoglycemia. Furthermore, the transplanted beads are difficult to retrieve, tend to be fragile, and will easily release islets upon slight damage.

A macroencapsulation procedure has also been tested. Macrocapsules of various different materials, such as poly-2-hydroxyethyl-methacrylate, polyvinylchloride-c-acrylic acid, and cellulose acetate were made for the immunoisolation of islets of Langerhans. (See Altman, et al., Diabetes 35: 625 (1986); Altman, et al., Transplantation: American Society of Artificial Internal Organs 30: 382 (1984); Ronel, et al., Jour. Biomedical Material Research 17: 855 (1983); Klomp, et al., Jour. Biomedical Material Research 17: 865-871 (1983)). In all these studies, only a transitory normalization of glycemia was achieved.

Archer et al., Journal of Surgical Research 28: 77 (1980), used acrylic copolymer hollow fibers to temporarily prevent rejection of islet xenografts. They reported long-term survival of dispersed neonatal murine pancreatic grafts in hollow fibers which were transplanted into diabetic hamsters. Recently Lacy et al., Science 254: 1782-1784 (1991) confirmed their results, but found the euglycemic state to be a transient phase. They found that when the islets are injected into the fiber, they aggregate within the hollow tube with resultant necrosis in the central portion of the islet masses. The central necrosis precluded prolongation of the graft. To solve this problem, they used alginate to disperse the islets in the fiber. However, this experiment has not been repeated extensively. Therefore, the membrane's function as an islet transplantation medium in humans is questionable.

Thus, there existed a need for achieving secretory cell transplantation, and, in particular, pancreatic islet allograft and xenograft survival without the use of chronic immunosuppressive agents.

In the Jain, et al work discussed *supra,* the inventors reported that encapsulating secretory cells in a hydrophilic gel material results in a functional, non-immunogenic material, that can be transplanted into animals and can be stored for long lengths of time. The encapsulation of the secretory cells provided a more effective and manageable technique for secretory cell transplantation. The encapsulation technique was described as being useful to encapsulate other biological agents, such as enzymes, microorganisms, trophic agents including recombinantly produced trophic agents, cytotoxic agents, and chemotherapeutic agents. The encapsulated biological agents were discussed as being useful to treat conditions known to respond to the biological agent.

The application does not discuss at any length the incorporation of cells which produce diffusible biological materials, the latter being useful in a therapeutic context. A distinction is made herein between secretory cells and cells which produce diffusible biological materials. The former, as per the examples given in the Jain application, refers generally to products such as hormones, cell signalling agents, etc., which are normally considered to be biological "messengers". In contrast, diffusible biological materials refers to materials such as MHC presented peptides, cell expression regulators such as suppressors, promoters, inducers, and so forth. The distinction will be seen in the field of oncology, e.g., as per the following discussion.

Extensive studies in cancer have included work on heterogeneous cell extracts, and various cellular components. Via the use of monoclonal antibodies, the art has identified relevant cancer associated antigens, e.g., GM2, TF, STn, MUC-1, and various epitopes derived therefrom. Current theory postulates that epitopes derived from these various tumor markers complex non-covalently, with MHC molecules, thereby forming an agrotype by specific cytolytic T cells. This mechanism is not unlike various mechanisms involved in the biological response to viral infections. Note in this regard, Van der Bruggen, et al., Science 254: 1643-1647 (1991); Boon, et al., 5,405,940, and Boon, et al., 5,342,774.

Additional research which parallels the work on identification of so-called cancer epitopes has focused on the regulation of cancer proliferation, such as via suppression or, more generally, biomodulation. See, e.g., Mitchell, J. Clin. Pharmacol 32: 2-9 1992); Maclean, et al., Can. J. Oncol. 4: 249-254 (1994). The aim which unites all of these diverse approaches to cancer is the modification of the host's immune response, so as to bring about some improvement in the patient's condition.

Key to all of these approaches is the activity of one or more diffusible biological products which act in concert with other materials to modulate the immune response. Boon, et al. and van der Bruggen, et al., e.g., disclose small peptide molecules. Mitchell discusses larger molecules which function, e.g., as suppressors.

One problem with all therapeutic approaches which employ these materials is the delivery of these in a safe, effective form. This is not easily accomplished. It has now been found, surprisingly, that the techniques of Jain, et al, which were so useful in the development of therapies for conditions requiring secretory cell products can now be used in other areas.

How this is accomplished is the subject of the invention, the detailed description of which follows.

### DETAILED DESCRIPTION OF PREFERED EMBODIMENTS

### Example 1

This example, and those which follow, employs RENCA cells. These are spontaneous renal adenocarcinoma cells of BALB/C mice, which are widely available, having been maintained in both *in vitro* and *in vivo* cultures. See Franco et al., Cytokine Induced Tumor Immunogenecity, 181-193 (1994).

Samples of frozen RENCA cells were thawed at 37°C, and then placed in tissue culture flasks containing Dulbecco's Modified Medium (D-MEM), which had been supplemented with 10% bovine serum, penicillin (100 u/ml) and streptomycin (50 ug/ml), to give what will be referred to as "complete medium" hereafter.

Cells were grown to confluency, and then trypsinized, followed by washing with Hank's Balanced Salt Solution, and then with the complete medium referred to *supra.*

In order to determine if the RENCA cells produced tumors efficiently, two BALB/C mice were injected, intraperitoneally, with 10° of these cells. The mice were observed, over a 3-4 week period. Clinically, they appeared healthy for the first two weeks, and exhibited normal activity. Thereafter, the clinical manifestations of cancer became evident. One mouse died after 23 days, and the second, after 25 days. Following death, the mice were examined, and numerous tumors of various size were noted. Some of the tumors exhibited hemorrhaging as well.

A sample of one tumor, taken from one of the mice, was fixed in 10% formalin for later histological examination.

### Example 2

Following the showing that the RENCA cells did grow *in vivo*, studies were carried out to determine if these cells grew in beads in accordance with the invention.

RENCA cells were grown to confluency, as described *supra,* trypsinized, and washed, also as described above. Samples of between 60,000 and 90,000 cells were then prepared. The cells were then centrifuged, at 750 RPMs, and fluid was removed. The cells were then suspended in solutions of 1% atelocollagen, in phosphate buffered saline solution, at a pH of 6.5.

A 1% solution of low viscosity agarose was prepared in minimal essential medium (MEM), maintained at 60°C, and then 100 µl of this were added to the suspension of RENCA cells and atellocollagen, described *supra.* The materials were then transferred, immediately, as a single large droplet, into sterile, room temperature mineral oil. The mixture formed a single, smooth, semi-solid bead. This procedure was repeated to produce a number of beads.

After one minute the beads were transferred to complete medium, as described *supra,* at 37°C. The beads were then washed three times in minimal essential medium containing the antibiotics listed *supra.* The beads were then incubated overnight at 37°C, in a humidified atmosphere of air and 5% CO₂. Following the incubation, the beads, now solid, were transferred to a sterile spoon which contained 1 ml of 5% agarose in minimal essential medium. Beads were rolled in the solution 2-3 times to uniformly coat them with agarose. The beads were transferred to mineral oil before the agarose solidified, to yield a smooth outer surface. After 60 seconds, the beads were washed, five times, with complete medium at 37°C to remove the oil. Overnight incubation (37°C, humidified atmosphere of air, 5% CO₂) followed.

These RENCA containing beads were used in the experiments which follow.

### Example 3

Prior to carrying out *in vivo* investigations, it was necessary to determine if the RENCA cells would grow in the beads prepared in the manner described *supra.*

To do this, beads prepared as discussed in example 2 were incubated in the medium described in example 2, for a period of three weeks under the listed conditions. Three of the beads were then cut into small pieces, and cultured in standard culture flasks, affording direct contact with both the flask and culture medium.

Observation of these cultures indicated that the cells grew and formed standard RENCA colonies. This indicated that the cells had remained viable in the beads.

### Example 4

*In vivo* experiments were then carried out. In these experiments, the beads were incubated for seven days, at 37°C. Subject mice then received bead transplants. To do this, each of four mice received a midline incision, carried through intraperitoneally. Three beads, each of which contained 60,000 RENCA cells were transplanted. Incisions were then closed (two layer closure), using an absorbable suture. The four mice (BALB/C) were normal, male mice, weighing between 24-26 grams, and appeared to be healthy. Two sets of controls were set up. In the first set, two mice received three beads containing no RENCA cells, and in the second, two mice were untreated with anything.

Three weeks after the implantation, all of the mice received intraperitoneal injections of 10⁶ RENCA cells. Eighteen days later, one control mouse died. All remaining mice were then sacrificed, and observed.

Control mice showed numerous tumors, while the mice which received the implants of bead-encapsulated cells showed only random nodules throughout the cavity.

These encouraging results suggested the design of the experiments set forth in the following example.

### Example 5

In these experiments, established cancers were simulated by injecting RENCA cells under one kidney capsule of each of six BALB/C mice. Fifteen days later, mice were divided into two groups. The three mice in the first group each received three beads, as described in example 4, *supra.* The second group (the control group) received beads which did not contain RENCA cells.

After 4-5 days, mice which had received RENCA cell containing implants looked lethargic, and their fur had become spiky, while the control group remained energetic, with no change in condition of fur.

Ten days after implantation (25 days after injection of RENCA cells), however, the control mice became sluggish, and exhibited distended abdomens. One of the three control mice died at fourteen days following bead transplantation. Sacrifice of the mice followed.

The body cavities of the control mice showed profuse hemorrhaging, with numerous tumors all over the alimentary canal, liver, stomach and lungs. The entire abdominal cavity had become indistinguishable due to rampant tumor growth. The mice which had received beads with encapsulated RENCA cells, however showed no hemorrhaging, and only a few nodules on the alimentary cancers. Comparison of test and control groups showed that, in the test group, nodules had not progressed.

### Example 6

Freely inoculated RENCA cell growth is inhibited when incubated along with encapsulated RENCA cells. A further set of experiments were carried out to determine if this effect was observable with other cells.

An adenocarcinoma cell line, i.e., MMT (mouse mammary tumor), was obtained from the American Type Culture Collection. Encapsulated MMT cells were prepared, as described, *supra* with MMT cells, to produce beads containing 120,000 or 240,000 cells per bead. Following preparation of the beads, they were used to determine if they would inhibit proliferation of RENCA cells *in vitro*. Specifically, two, six well petri plates were prepared, via inoculation with 1x10⁴ RENCA cells per well, in 4 ml of medium. In each plate, three wells served as control, and three as test. One of the three control wells in each plate received one bead. Each of the other wells received either two or three empty beads. The second well was treated similarly, with wells receiving one, two or three beads containing 120,000 or 240,000 MMT cells. Wells were incubated at 37°C for one week, after which RENCA cells were trypsinized, washed, and counted, using a hemocytometer. Results follow:

| DISH # 1 (EMPTY MACROBEADS) # of cells retrieved after one week | | | DISH # 2 (MACROBEADS WITH MMT CELLS) # of cells retrieved after one week | |
|---|---|---|---|---|
| Well # | Control | Empty | 120,000 MMT cells | 240,000 MMT cells |
| 1 | 2.4 x 10⁵ | 2.8 x 10⁵ | 1.4 x 10⁵ | 1 x 10⁵ |
| 2 | 2.0 x 10⁵ | 3.5 x 10⁵ | 1.2 x 10⁵ | 7 x 10⁴ |
| 3 | 4.4x10⁵ | 2.5x10⁵ | 1.25x10⁵ | 9 x 10⁴ |

### Example 7

Following the results in example 6, the same experiments was carried out, using 1x10⁴ MMT cells rather than RENCA cells. The experiment was carried out precisely as example 6. Results are set forth below.

| Well # | Control | Empty Macrobeads | (1) MMT Macrobeads | (2) MMT Macrobeads |
|---|---|---|---|---|
| 1 | 3.1x10⁶ | 2.8x10⁶ | 1.6 x 10⁶ | 1.3 x 10⁶ |
| 2 | 3.3 x 10⁶ | 2.6. x 10⁶ | 1.0 x 10⁶ | 1.1 x 10⁶ |
| 3 | 3.0 x 10⁶ | 2.8 x 10⁶ | 6.0 x 10⁵ | 5.0 x 10⁵ |
| | | | | |

These results encouraged the use of an *in vivo* experiment. This is presented in example 8.

### Example 8

RENCA cells, as used in the preceding examples, are renal cancer cells. To demonstrate more completely the general efficacy of the invention, work was carried out using a different type of cancer cells. Specifically, adenocarcinoma cells were used.

A mouse mammary tumor cell line (MMT) was obtained from the American Type Culture Collection. Using the protocols set forth, *supra,* implants were prepared which contained 120,000 cells per bead, and 240,000 cells per bead.

The experimental model used was the mouse model, *supra.* Twenty two mice were divided into groups of 4, 9 and 9. The first group, i.e., the controls, were further divided into three groups of two, one and one. The first subgroup received implants of one bead containing no cells. One mouse received two empty beads, and one received three empty beads.

Within experimental group A (9 animals), the beads contained 120,000 cells, while in group B, the beads contained 240,000 cells. Within groups "A" and "B", there were three subdivisions, each of which contained three mice. The subgroups received one, two, or three beads containing MMT cells.

Twenty one days following implantation, all animals received injections of 40,000 REMCA cells. Immediately after injection, the mice were lethargic, with spiky hair. This persisted for about five days, after which normal behavior was observed.

After twenty days, control mice exhibited distended abdomens, and extremely spiky hair. One control mouse died 25 days following injection, while the remaining control mice appeared terminal. All mice were sacrificed, and tumor development was observed. These observations are recorded infra:

| NUMBER OF MACROBEADS IN MICE | CONTROL | EXPERIMENTAL GROUP A | EXPERIMENTAL GROUP B |
|---|---|---|---|
| 1 | ++++ | -- | -- |
| 1 | ++++ | -- | -- |
| 1 | | + | ++ |
| | | | |
| 2 | ++++ | -- | -- |
| 2 | | -- | -- |
| 2 | | ++ | ++ |
| | | | |
| 3 | ++++ | -- | -- |
| 3 | | -- | -- |
| 3 | | -- | +++ |

These results show that, of eighteen mice tested, thirteen showed no disease. Of the mice in Group (A), one mouse exhibited a few modules, and another mouse showed a few tumors. One mouse which received two beads showed a few tumors.

Within group B, one mouse which had received one bead, and one mouse which received two beads showed a few tumors, entangled with intestines. One of the mice which received three beads had developed a large solid tumor and was apparently very sick. Nonetheless, the overall results showed that the encapsulated mouse mammary tumor cells inhibited tumor formation.

### Example 9

As suggested, *supra,* the practice of the invention results in the production of some material or factor which inhibits and/or prevents tumor cell proliferation. This was explored further in the experiment which follows.

Additional beads were made, as described *supra* in example 2, except that atellocallogen was not included. Hence, these beads are agarose/agarose beads. RENCA cells, as described, *supra,* were incorporated into these beads, again as described *supra.*

Two sets of three six well plates were then used as control, and experimental groups. In the control group, wells were filled with 4 ml of RPMI complete medium (10% fetal calf serum and 11 ml/l of penicillin). Each control group well was then inoculated with 10,000 RENCA cells.

In the experimental group, the RPMI complete medium was conditioned, by adding material secured by incubating 10 immuno-isolated, RENCA containing beads (120,000 cell per bead), in a 35x100 mm petri plate containing 50 ml of the RPMI complete medium. Following five days of incubation, medium was collected from these plates, and 4 ml of it was placed in each test well. These wells were then inoculated with 10,000 RENCA cells.

All plates (both control and experimental) were incubated at 37°C for five days. Following the incubation period cells were trypsinization washed, and counted using a hemocytometer. The cells in the plates of each well were pooled together following trypsinization, and counted, the results follow.

| WELL # | (CELLS) CONTROL | (CELLS) CONDITIONED |
|---|---|---|
| 1 | 7 x 10⁵ | 3 x 10⁵ |
| 2 | 8 x 10⁵ | 2.5 x 10⁵ |
| 3 | 7 x 10³ | 3.4 X 10⁵ |

These results show that the cells, when restricted in, e.g., the beads of the invention, produced some factor which resulted in suppression of tumor cell proliferation. This restriction inhibitor factor is produced by the cells in view of their entrapment in the bead, and differs from other materials such as contact inhibitor factor, which are produced when cells contact each other.

### Example 10

The experiment set forth *supra* showed that RENCA cell growth, in conditioned medium, was about half the growth of the cells in control medium. The experiments set forth herein examined whether the growth inhibiting factor would remain active after the conditioned medium was frozen.

RENCA conditioned medium was prepared by incubating 10 immunoisolated RENCA containing beads for five days. Incubation was in 35x100 mm petri plates, with 50 ml RMPI complete medium, at 37°C. Following the incubation, the medium was collected and stored at -20°C. Conditioned medium was prepared by incubating immunoisolated MMT (mouse mammary tumor) cell containing beads. The beads contained 240,000 cell per bead; otherwise all conditions were the same.

Frozen media were thawed at 37°C, and then used in the following tests. Three six well plates were used for each treatment, i.e., (i) RMPI control medium, (2) RENCA frozen conditioned medium, and (3) MMT frozen conditioned medium. A total of 4 ml of medium was dispensed into each well. All wells were then inoculated with 10,000 RENCA cells, and incubated at 37°C, for five days. Following incubation, two plates of samples were taken from each well, trypsinized, pooled, and counted in a hemocytometer. At eight days, the remaining three plates of each well were tested in the same way.

Results follow:

| DISH 5 DAYS OLD | CONTROL MEDIUM | FROZEN CONDITIONED MEDIUM OF RENCA | FROZEN CONDITIONED MEDIUM OF MMT |
|---|---|---|---|
| 1 | 6 x 10⁵ | 5 X 10⁵ | 8 X 10⁴ |
| 2 | 6.8 X 10⁵ | 4.2 X 10⁵ | 8.5 x 10⁴ |
| 8 DAYS OLD | | | |
| 3 | 2.8 X 10⁶ | 2 X 10⁶ | 8 X 10⁴ |

When these results are compared to those in example 6, supra, it will be seen that, while the frozen/thawed RENCA conditioned medium did not arrest growth to the same extent that unfrozen medium did (compare examples 6 and 7), it did, nonetheless, arrest growth). Frozen conditioned medium using MMT cells arrested growth even more than the unfrozen MMT conditioned medium. These results show that, of eighteeen mice tested, thirteen showed no disease. Of the mice in Group (A), one mouse exhibited a few modules, and another mouse showed a few tumors.

The foregoing describes the manufacture of implantable beads which contain one or more types of cells which produce a diffusible biological product, as this phrase is defined herein. The diffusible biological product is one which has an effect on the subject in which the bead is implanted. Preferably, this effect is immunomodulation, such as stimulating an immune response, or suppressing a response. In the case of cancer, for example, the diffusible biological product may be a peptide which complexes with MHC molecules on cancer cells in a subject, thereby provoking a CTL response thereto in turn leading to lowering of the tumor load in the subject. The diffusible product may also be a suppressor of tumor growth. In connection with this form of therapy, it is possible, although not necessarily preferable, to place the implanted beads in or near an identified tumor.

"Diffusible biological product" as used herein refers to materials such as proteins, glycoproteins, lipoproteins, carbohydrates, lipids, glycolipids, and peptides. More specifically, materials such as antibodies, cytokines, hormones, enzymes, and so forth, are exemplary, but by no means the only type of materials included. Excluded are the well known "end products" of cellular processes, such as CO₂ and H₂O.

As the experiments show, the implantable beads may also be used prophylactically. It is well known that at least a segment of the population of cancer patients are prone to reoccurrence of the condition. The experiments described herein show that the implants can prevent the occurrence or reoccurrence of cancer, via the biological effect the diffusible product has on a subject's system.

The discussion of the invention has focused on *in vivo* approaches. It must also be understood that there are *in vitro* approaches to the invention, some of which are discussed upon herein. For example, it is well known that many cells which produce desirable products, when cultured *in vitro,* require the presence of feeder cells. There are always issues with such feeder cells. They may grow faster than the desired cells, leading to de facto "strangulation" of the materials of interest. Further, there can be a problem with various toxic products being produced by the feeder layers. The implantable beads of the invention act almost as cellular incubators, protecting the incorporated cells, while permitting the diffusible products to move into a culture medium, e.g., where they can be collected.

As indicated, *supra,* preparation of the implantable beads first requires suspension of the cells in solution, preferably aqueous of collagen. Preferably, the collagen is atelocollagen, in a solution of from about 0.5 to about 2%. Depending upon the type of cell used, the number of cells in the solution at a given time, and hence the number of cells in a bead, will vary. Preferably, there are from about 10,000 to about 200,000 cells used per bead, more preferably from about 30,000 to about 100,000. Most preferably, about 40,000 to about 60,000 cells are used.

Following suspension of the cells in the collagen solution, an agarose solution is added. Preferably, this agarose solution will range from about 0.5% to about 5%, preferably about 1%. By dropping the mixture onto or into inert materials, such as TEFLON^{®} or mineral oil, a bead forms. This bead is semi-solid. The semi-solid bead is then transferred to a sterile medium, preferably one containing antibiotics, washed, and incubated to polymerize collagen. The polymerization of collagen is a well studied phenomenon, and the conditions under which this occurs need not be elaborated upon herein.

Following the solidification of the bead, it is then coated with agarose, preferably by rolling it in an agarose solution. One preferred way of accomplishing this is a simple TEFLON^{®} coated spoon which contains a solution of agarose, preferably 5% to 10%.

The foregoing discussion of diffusible biological products should not be construed as being limited to wild type materials. For example, one can just as easily incorporate transformed or transfected host cells, such as eukaryotic cells (e.g., 293 cells, CHO cells, COS cells), or even prokaryotic cells (e.g., E. coli), which have been treated to produce heterogeneous protein, or modified via, e.g., homologous recombination, to produce increased amounts of desirable biological products, Other materials, such as hybridomas, may also be used, with the diffusible biological product being a monoclonal antibody.

Other features and aspects of the invention will be clear to the skilled artisan, and need not be related here.

## Claims

1. An agarose coated, agarose-containing bead which contains proliferation suppressing material producing cancer cells.

2. The bead according to claim 1, wherein said cell is a renal cancer cell or a breast cancer cell.

3. The bead according to claim 1 or 2 comprising preferably from about 10.000 to about 200.000 cells, more preferably from about 30.000 to about 100.000 cells, most preferably from about 40.000 to about 60.000 cells.

4. Method of producing an agarose coated, agarose-containing bead according to any of claims 1 to 3 comprising:
(a) suspending proliferation suppressing material producing cells in a solution of agarose,
(b) forming a smooth semi-solid bead from said suspended cells of step (a),
(c) incubating said semi-solid bead of step (b) while forming a solid bead,
(d) coating said solid bead of step (c) with agarose to obtain an agarose coated, agarose-containing bead.

5. Method of claim 4, wherein step (a) comprises adding a 1% solution of low viscosity agarose to the cells and/or step (d) comprises rolling said solid bead of step (c) in 5% agarose, and/or contacting said rolled solid bead to mineral oil, and/or washing said rolled bead to obtain said agarose coated, agarose-containing bead.

6. Method of claim 4 or 5, wherein said cell is a renal cancer cell or a breast cancer cell.

7. Use of an agarose coated, agarose-containing bead according to any of claims 1 to 3 for the preparation of a medicament for suppressing proliferation of cells in a subject in need thereof.

8. Use of the beads of any of claims 1 to 3 for the production of a diffusible biological product.

9. The use of claim 8, wherein a therapeutically effective amount of the product is employed for the preparation of a medicament for suppressing proliferation of cells in a subject in need thereof.

10. Method of producing a conditioned medium comprising
incubating at least one bead of any of claims 1 to 3 in medium, wherein a material diffuses through said bead into the medium,
under appropriate conditions, and
collecting the medium with appropriate means.

11. Use of the beads of any of claims 1-3 for the production of a conditioned medium.

12. The use of claim 11, wherein the cells are renal cancer cells or breast cancer cells.

13. The use of claim 11 or 12, wherein the cells are mouse or human.

14. The use of any of claims 11 to 13, wherein the bead contains from about 10.000 to about 200,000 cells and, preferably from about 30.000 to about 100.000 cells, most preferably from about 40.000 to about 60.000 cells.

15. The use of any of claims 11 to 14, wherein the beads are incubated in RPMI complete medium for five days.

16. The use of any one of claims 11 to 15, wherein the conditioned medium is frozen.

17. The use of claims 11 to 16, wherein the conditioned medium contains material which is capable of cell proliferation suppression.

18. The use of claim 17, wherein the material is a protein, glycoprotein, lipoprotein, carbohydrate, lipid, glycolipid, peptide, antibody, cytokine, hormone or enzyme.

19. The use of any of claims 11 to 18, wherein the conditioned medium or the material is for the preparation of a medicament for stimulating, or suppressing an immune response, or for the treatment of a pathological condition which is **characterized by** abnormal cell growth in a subject.

20. Use according to claim 19, wherein the pathological condition is cancer.

21. The use of any of claims 11 to 18, wherein the conditioned medium or the material is for *in vitro* applications such as research, testing and diagnosis.

22. Use according to claim 19 or 20, wherein said restricted or entrapped cells are of the same type associated with the condition with which said subject is afflicted, or of a type different from the type of cell associated with the condition with which said subject is afflicted.

23. Use according to claim 19 or 20, wherein said restricted or entrapped cells are cells taken from the subject to which said composition of matter is administered.

24. A method for determining if a composition of matter is useful in suppressing proliferation of cancer cells, comprising
(a) adding a sample of non-entrapped cancer cells to a culture medium,
wherein said culture medium has been used to culture a composition of matter comprising a sample of cancer cells entrapped in a bead of any of claims 1 to 3,
culturing said sample of non-entrapped cancer cells in said culture medium for a defined period of time and
comparing proliferation of said non-entrapped cancer cells to a sample of said non-entrapped cancer cells in the same culture medium, wherein said culture medium has not been used to culture said composition of matter,
wherein a decrease in proliferation indicates that said composition of matter is useful in suppressing proliferation of said non-entrapped cancer cells, or
(b) mixing a sample of non-entrapped cancer cells, culture medium, and a composition of matter comprising a sample of cancer cells entrapped in a bead of any of claims 1 to 3 to form a first mixture,
culturing said mixture for a defined period of time, and comparing proliferation of said non-entrapped cancer cells to a second mixture of said culture medium and said non-entrapped cancer cells, in the absence of said composition of matter, cultured for the same period of time, wherein a decrease in proliferation in said first mixture indicates that said composition of matter is useful in suppressing proliferation of said non-entrapped cancer cells.

25. The method of claim 24, wherein said entrapped cancer cells are breast cancer cells or renal cancer cells.

26. The method of claim 24, wherein said entrapped and/or non-entrapped cancer cells are human cancer cells, or mouse cancer cells.

27. The method of claim 24, wherein said composition of matter contains preferably from about 10.000 to about 200.000 cells, more preferably from about 30.000 to about 100.000 cells, most preferably from about 40.000 to about 60.000 cells.

28. The method of claim 24, wherein said entrapped cancer cells are of the same type or are of a different type as the non-entrapped cancer cells.

## Patentansprüche

1. Agarose-beschichtetes, Agarose-enthaltendes Kügelchen, das Krebszellen enthält, die Proliferation-supprimierendes Material erzeugen.

2. Kügelchen nach Anspruch 1, worin die Zelle eine Nierenkrebszelle oder eine Brustkrebszelle ist.

3. Kügelchen nach Anspruch 1 oder 2, umfassend vorzugsweise von etwa 10.000 bis etwa 200.000 Zellen, mehr bevorzugt von etwa 30.000 bis etwa 100.000 Zellen, am meisten bevorzugt von etwa 40.000 bis etwa 60.000 Zellen.

4. Verfahren zum Herstellen eines Agarose-beschichteten, Agarose-enthaltenden Kügelchens nach einem der Ansprüche 1 bis 3, umfassend
(a) Suspendieren von Proliferation-supprimierendem Material-erzeugenden Zellen in einer Lösung aus Agarose,
(b) Bilden eines glatten semi-festen Kügelchens aus den suspendierten Zellen von Schritt (a),
(c) Inkubieren des semi-festen Kügelchens von Schritt (b) unter Bildung eines festen Kügelchens;
(d) Beschichten des festen Kügelchens von Schritt (c) mit Agarose, um ein Agarose-beschichtetes, Agarose-enthaltendes Kügelchen zu erhalten.

5. Verfahren nach Anspruch 4, worin Schritt (a) Zugeben einer 1 %-igen Lösung von Agarose mit geringer Viskosität zu den Zellen und/oder Schritt (d) das Rollen des festen Kügelchens aus Schritt (c) in 5 %-iger Agarose und/oder In-Kontakt-bringen des gerollten festen Kügelchens mit Mineralöl und/oder Waschen des gerollten Kügelchens, um das Agarose-beschichtete, Agarose-enthaltende-Kügelchen zu erhalten, umfasst.

6. Verfahren nach Anspruch 4 oder 5, worin die Zelle eine Nierenkrebszelle oder eine Brustkrebszelle ist.

7. Verwendung eines Agarose-beschichteten, Agarose-enthaltenden Kügelchens nach einem der Ansprüche 1 bis 3 zur Herstellung eines Medikaments zum Supprimieren der Proliferation von Zellen in einem Individuum, das dies benötigt.

8. Verwendung der Kügelchen nach einem der Ansprüche 1 bis 3 zur Herstellung eines diffusionsfähigen biologischen Produkts.

9. Verwendung nach Anspruch 8, worin eine therapeutisch wirksame Menge des Produkts zur Herstellung eines Medikaments zur Unterdrückung der Proliferation von Zellen in einem Individuum, das dies benötigt, verwendet wird.

10. Verfahren zum Herstellen eines konditionierten Mediums, umfassend mindestens ein Kügelchen nach einem der Ansprüche 1 bis 3 in Medium,
worin ein Material durch das Kügelchen in das Medium unter geeigneten Bedingungen diffundiert und Aufnehmen des Mediums mit geeigneten Mitteln.

11. Verwendung der Kügelchen nach einem der Ansprüche 1-3 zur Herstellung eines konditionierten Mediums.

12. Verwendung nach Anspruch 11, worin die Zellen Nierenkrebszellen oder Brustkrebszellen sind.

13. Verwendung nach Anspruch 11 oder 12, worin die Zellen aus Maus oder aus Mensch sind.

14. Verwendung nach einem der Ansprüche 11 bis 13, worin das Kügelchen von etwa 10.000 bis etwa 200.000 Zellen, mehr bevorzugt von etwa 30.000 bis etwa 100.000 Zellen, am meisten bevorzugt von etwa 40.000 bis etwa 60.000 Zellen enthält.

15. Verwendung nach einem der Ansprüche 11 bis 14, worin die Kügelchen in RPMI-Komplettmedium für fünf Tage inkubiert werden.

16. Verwendung nach einem der Ansprüche 11 bis 15, worin das konditionierte Medium gefroren ist.

17. Verwendung nach einem der Ansprüche 11 bis 16, worin das konditionierte Medium Material enthält, das in der Lage zur Zellproliferationssuppression ist.

18. Verwendung nach Anspruch 17, worin das Material ein Protein, Glycoprotein, Lipoprotein, Kohlenhydrat, Lipid, Glycolipid, Peptid, Antikörper, Cytokin, Hormon oder Enzym ist.

19. Verwendung nach einem der Ansprüche 11 bis 18, worin das konditionierte Medium oder das Material zur Herstellung eines Medikaments zum Stimulieren oder Supprimieren einer Immunantwort oder zur Behandlung eines pathologischen Zustands, der **gekennzeichnet ist durch** anomales Zellwachstum, in einem Individuum, dient.

20. Verwendung nach Anspruch 19, worin der pathologische Zustand Krebs ist.

21. Verwendung nach einem der Ansprüche 11 bis 18, worin das konditionierte Medium oder das Material für in vitro-Anwendungen, wie etwa Forschung, Testen und Diagnose ist.

22. Verwendung nach Anspruch 19 oder 20, worin die restringierten oder eingefangenen Zellen vom gleichen Typ sind, wie demjenigen, der mit dem Zustand verbunden ist, von welchem das Lebewesen betroffen ist, oder von einem Typ, der verschieden ist von dem Zelltyp, der mit dem Zustand assoziiert ist von dem das Individuum betroffen ist.

23. Verwendung nach Anspruch 19 oder 20, worin die restringierten oder eingefangenen Zellen Zellen sind, die aus dem Individuum entnommen wurden, welchem die Stoffzusammensetzung verabreicht wird.

24. Verfahren zum Bestimmen ob eine Stoffzusammensetzung geeignet ist zum Supprimieren von Proliferation von Krebszellen, umfassend
(a) Zugeben einer Probe von nichteingefangenen Krebszellen zu einem Kulturmedium, worin das Kulturmedium verwendet worden ist zum Kultivieren einer Stoffzusammensetzung, umfassend eine Probe von Krebszellen, die eingefangen sind in einem Kügelchen nach einem der Ansprüche 1 bis 3,
Kultivieren der Probe von nichteingefangenen Krebszellen in dem Kulturmedium für eine definierte Zeitdauer und
Vergleichen der Proliferation der nichteingefangenen Krebszellen mit einer Probe der nichteingefangenen Krebszellen im gleichen Kulturmedium, worin das Kulturmedium nicht verwendet worden ist zum Kultivieren der
Stoffzusammensetzung,
worin eine Abnahme der Proliferation anzeigt, dass die Stoffzusammensetzung geeignet ist zum Supprimieren von Proliferation von nichteingefangenen Krebszellen, oder
(b) Mischen einer Probe von nichteingefangenen Krebszellen, Kulturmedium und einer Stoffzusammensetzung, umfassend eine Probe von Krebszellen, eingefangen in einem Kügelchen nach einem der Ansprüche 1 bis 3, um ein erstes Gemisch zu bilden,
Kultivieren des Gemischs für eine definierte Zeitdauer und Vergleichen der Proliferation der nichteingefangenen Krebszellen mit einem zweiten Gemisch des Kulturmediums und der nichteingefangenen Krebszellen, in der Abwesenheit der Stoffzusammensetzung, kultiviert für die gleiche Zeitdauer,
worin eine Abnahme der Proliferation in dem ersten Gemisch anzeigt, dass die Stoffzusammensetzung geeignet ist zum Supprimieren der Proliferation der nichteingfangenen Krebszellen.

25. Verfahren nach Anspruch 24, worin die eingefangenen Krebszellen Brustkrebszellen oder Nierenkrebszellen sind.

26. Verfahren nach Anspruch 24, worin die eingefangenen und/oder nichteingefangenen Krebszellen Krebszellen aus Mensch oder Krebszellen aus Maus sind.

27. Verfahren nach Anspruch 24, worin die Stoffzusammensetzung vorzugsweise von etwa 10.000 bis etwa 200.000 Zellen, mehr bevorzugt von etwa 30.000 bis etwa 100.000 Zellen, am meisten bevorzugt von etwa 40.000 bis etwa 60.000 Zellen enthält.

28. Verfahren nach Anspruch 24, worin die eingefangenen Krebszellen vom gleichen Typ sind oder von einem verschiedenen Typ sind wie die nichteingefangenen Krebszellen.

## Revendications

1. Bille contenant de l'agarose et recouverte d'agarose contenant des cellules cancéreuses produisant une substance inhibant la prolifération.

2. Bille selon la revendication 1, dans laquelle ladite cellule est une cellule de cancer du rein ou une cellule de cancer du sein.

3. Bille selon la revendication 1 ou 2, comprenant de préférence environ 10 000 à environ 200 000 cellules, et de manière davantage préférée environ 30 000 à environ 100 000 cellules, et de manière préférée entre toutes environ 40 000 à environ 60 000 cellules.

4. Procédé de production d'une bille contenant de l'agarose et recouverte d'agarose selon l'une quelconque des revendications 1 à 3, comprenant :
(a) la suspension de cellules produisant une substance inhibant la prolifération dans une solution d'agarose,
(b) la formation d'une bille semi-solide lisse à partir desdites cellules suspendues de l'étape (a),
(c) l'incubation de ladite bille semi-solide de l'étape (b) tout en formant une bille solide,
(d) le revêtement de ladite bille solide de l'étape (c) avec de l'agarose pour obtenir une bille contenant de l'agarose et recouverte d'agarose.

5. Procédé selon la revendication 4, dans lequel l'étape (a) comprend l'ajout d'une solution à 1 % d'agarose de basse viscosité aux cellules et/ou l'étape (d) comprend le roulage de ladite bille solide de l'étape (c) dans de l'agarose à 5 %, et/ou la mise en contact de ladite bille solide roulée avec une huile minérale, et/ou le lavage de ladite bille roulée pour obtenir ladite bille contenant de l'agarose et recouverte d'agarose.

6. Procédé selon la revendication 4 ou 5, dans lequel ladite cellule est une cellule de cancer du rein ou une cellule de cancer du sein.

7. Utilisation d'une bille contenant de l'agarose et recouverte d'agarose selon l'une quelconque des revendications 1 à 3, pour la préparation d'un médicament destiné à inhiber la prolifération de cellules chez un sujet en ayant besoin.

8. Utilisation des billes selon l'une quelconque des revendications 1 à 3, pour la production d'un produit biologique diffusable.

9. Utilisation selon la revendication 8, dans laquelle une quantité efficace d'un point de vue thérapeutique du produit est utilisée pour la préparation d'un médicament destiné à inhiber la prolifération de cellules chez un sujet en ayant besoin.

10. Procédé de production d'un milieu conditionné comprenant
l'incubation d'au moins une bille selon l'une quelconque des revendications 1 à 3 dans un milieu,
dans lequel une substance se diffuse à travers ladite bille dans le milieu,
dans des conditions appropriées, et
la récupération du milieu avec un moyen approprié.

11. Utilisation des billes selon l'une quelconque des revendications 1 à 3, pour la production d'un milieu conditionné.

12. Utilisation selon 1-a revendication 11, dans laquelle les cellules sont des cellules de cancer du rein ou des cellules de cancer du sein.

13. Utilisation selon la revendication 11 ou 12, dans laquelle les cellules sont des cellules de souris ou d'humain.

14. Utilisation selon l'une quelconque des revendications 11 à 13, dans laquelle la bille contient environ 10 000 à environ 200 000 cellules et, de préférence environ 30 000 à environ 100 000 cellules, et de manière préférée entre toutes environ 40 000 à environ 60 000 cellules.

15. Utilisation selon l'une quelconque des revendications 11 à 14, dans laquelle les billes sont incubées dans un milieu RPMI complet pendant cinq jours.

16. Utilisation selon l'une quelconque des revendications 11 à 15, dans laquelle le milieu conditionné est congelé.

17. Utilisation selon les revendications 11 à 16, dans laquelle le milieu conditionné contient une substance qui est capable d'inhiber la prolifération des cellules.

18. Utilisation selon la revendication 17, dans laquelle la substance est une protéine, une glycoprotéine, une lipoprotéine, un glucide, un lipide, un glycolipide, un peptide, un anticorps, une cytokine, une hormone ou une enzyme.

19. Utilisation selon l'une quelconque des revendications 11 à 18, dans laquelle le milieu conditionné ou la substance est utilisé (e) pour préparer un médicament permettant de stimuler ou d'inhiber une réponse immunitaire, ou destiné au traitement d'un état pathologique **caractérisé par** une croissance cellulaire anormale chez un sujet.

20. Utilisation selon la revendication 19, dans laquelle l'état pathologique est le cancer.

21. Utilisation selon les revendications 11 à 18, dans laquelle le milieu conditionné ou la substance est utilisé(e) pour des applications *in vitro,* telles que la recherche, des tests et des diagnostics.

22. Utilisation selon la revendication 19 ou 20, dans laquelle lesdites cellules enfermées ou piégées sont du même type que celles associées à la maladie dont souffre ledit sujet, ou d'un type différent du type de cellules associées à la maladie dont souffre ledit sujet.

23. Utilisation selon la revendication 19 ou 20, dans laquelle lesdites cellules enfermées ou piégées sont des cellules prélevées sur le sujet auquel ladite composition de matière est administrée.

24. Procédé permettant de déterminer si une composition de matière est utile pour inhiber la prolifération de cellules cancéreuses, comprenant
(a) l'ajout d'un échantillon de cellules cancéreuses non-piégées à un milieu de culture, dans lequel ledit milieu de culture a été utilisé pour réaliser la culture d'une composition de matière comprenant un échantillon de cellules cancéreuses piégées dans une bille selon l'une quelconque revendications 1 à 3, la culture dudit échantillon de cellules cancéreuses non-piégées dans ledit milieu de culture pendant une période de temps définie et
la comparaison entre la prolifération desdites cellules cancéreuses non-piégées et la prolifération d'un échantillon desdites cellules cancéreuses non-piégées dans le même milieu de culture, dans laquelle ledit milieu de culture n'a pas été utilisé pour réaliser la culture de ladite composition de matière,
dans lequel une diminution de la prolifération indique que ladite composition de matière est utile pour inhiber la prolifération desdites cellules cancéreuses non-piégées, ou
(b) le mélange d'un échantillon de cellules cancéreuses non-piégées, d'un milieu de culture et d'une composition de matière comprenant un échantillon de cellules cancéreuses piégées dans une bille selon l'une quelconque des revendications 1 à 3 pour former un premier mélange,
la culture dudit mélange pendant une période de temps définie et la comparaison entre la prolifération desdites cellules cancéreuses non-piégées et la prolifération d'un second mélange dudit milieu de culture et desdites cellules cancéreuses non-piégées, en absence de ladite composition de matière et dont la culture a été réalisée pendant la même période de temps, où une diminution de la prolifération dans ledit premier mélange indique que ladite composition de matière est utile pour inhiber la prolifération desdites cellules cancéreuses non-piégées.

25. Procédé selon la revendication 24, dans lequel les dites cellules cancéreuses piégées sont des cellules de cancer du sein ou des cellules de cancer du rein.

26. Procédé selon la revendication 24, dans lequel lesdites cellules cancéreuses piégées et/ou non-piégées sont des cellules cancéreuses humaines ou des cellules cancéreuses murines.

27. Procédé selon la revendication 24, dans lequel ladite composition de matière contient de préférence environ 10 000 à environ 200 000 cellules, et de manière davantage préférée environ 30 000 à environ 100 000 cellules, et de manière préférée entre toutes environ 40 000 à environ 60 000 cellules.

28. Procédé selon la revendication 24, dans lequel lesdites cellules cancéreuses piégées sont du même type ou sont d'un type différent de celui des cellules cancéreuses non-piégées.
